(19) **Europäisches Patentamt**
**European Patent Office**
**Office européen des brevets**

(11) **EP 2 150 253 B1**

(12) **FASCICULE DE BREVET EUROPEEN**

(45) Date de publication et mention
de la délivrance du brevet:
**30.03.2011   Bulletin 2011/13**

(21) Numéro de dépôt: **08787962.3**

(22) Date de dépôt: **16.04.2008**

(51) Int Cl.:
*A61K 31/444* *(2006.01)*        *A61P 25/00* *(2006.01)*

(86) Numéro de dépôt international:
**PCT/FR2008/000533**

(87) Numéro de publication internationale:
**WO 2008/145840 (04.12.2008 Gazette 2008/49)**

(54) **UTILISATION DU 4-CYCLOPROPYLMETHOXY-N-(3,5-DICHLORO-1 -OXYDO- PYRIDIN-4-YL)-5-(METHOXY)PYRIDINE-2-CARBOXAMIDE POUR LE TRAITEMENT DES TRAUMATISMES DE LA MOELLE EPINIERE**

VERWENDUNG VON 4-CYCLOPROPYLMETHOXY-N-(3,5-DICHLOR-1-OXIDOPYRIDIN-4-YL)-5-(METHOXY)-PYRIDIN-2-CARBOXAMID ZUR BEHANDLUNG VON RÜCKENMARKSVERLETZUNGEN

USE OF 4-CYCLOPROPYLMETHOXY-N-(3,5-DICHLORO-1-OXIDOPYRIDIN-4-YL)-5-(METHOXY) PYRIDINE-2-CARBOXAMIDE FOR THE TREATMENT OF SPINAL CORD TRAUMAS

(84) Etats contractants désignés:
**AT BE BG CH CY CZ DE DK EE ES FI FR GB GR HR HU IE IS IT LI LT LU LV MC MT NL NO PL PT RO SE SI SK TR**
Etats d'extension désignés:
**AL BA MK RS**

(30) Priorité: **19.04.2007   FR 0702851**

(43) Date de publication de la demande:
**10.02.2010   Bulletin 2010/06**

(73) Titulaire: **Sanofi-Aventis
75013 Paris (FR)**

(72) Inventeurs:
• **DELAY-GOYET, Philippe
F-75013 Paris (FR)**
• **FERZAZ, Badia
F-75013 Paris (FR)**
• **LOLIVIER, Jocelyne
F-75013 Paris (FR)**

(74) Mandataire: **Gaslonde, Aude et al
Sanofi-Aventis
Département Brevets
174, Avenue de France
75013 Paris (FR)**

(56) Documents cités:
**WO-A-95/04045        GB-A- 2 406 856
US-A1- 2007 021 451**

• **NIKULINA E ET AL: "The phosphodiesterase inhibitor rolipram delivered after a spinal cord lesion promotes axonal regeneration and functional recovery" PROCEEDINGS OF THE NATIONAL ACADEMY OF SCIENCES OF THE UNITED STATES OF AMERICA 08 JUN 2004 UNITED STATES, vol. 101, no. 23, 8 juin 2004 (2004-06-08), pages 8786-8790, XP007903694 ISSN: 0027-8424**

Printed by Jouve, 75001 PARIS (FR)

**EP 2 150 253 B1**

**Description**

La présente invention a pour objet l'utilisation du 4-cyclopropylméthoxy-*N*-(3,5-dichloro-1-oxydo-pyridin-4-yl)-5-(méthoxy)pyridine-2-carboxamide sous forme d'hydrate, de solvate, de base ou de sel d'addition à un acide, pour la préparation d'un médicament destiné au traitement des traumatismes de la moelle épinière.

Le 4-cyclopropylméthoxy-*N*-(3,5-dichloro-1-oxydo-pyridin-4-yl)-5-(méthoxy)pyridine-2-carboxamide ou encore appelé *N*-(3,5-dichloro-1-oxydo-4-pyridinio)-4-cyclopropylméthoxy-5-méthoxypyridine-2-carboxamide, est connu pour entrer dans la composition de médicaments destinés au traitement de différentes pathologies dont notamment les inflammations articulaires, l'arthrite, l'arthrite rhumatoïde. Ce composé, sous forme hémihydrate, est décrit par exemple dans le document WO95/04045 (composé référencé FR).

GB-A-2 406 856 décrit l'utilisation de composés du type pyridine-CO-NH-pyridine pour le traitement de blessures de la moelle épinière.

US 2007/021451 décrit l'utilisation de dérivés de la pyridine pour traiter des problèmes neurologiques survenus après une blessure à la moelle épinière.

L'article "The phosphodiesterase inhibitor rolipram delivered after a spinal cord lesion promotes axonal regeneration and functional recovery" (Nikulina Elena et al.; PROCEEDINGS OF THE NATIONAL ACADEMY OF SCIENCES OF THE UNITED STATES OF AMERICA 08 JUN 2004 UNITED STATES, vol. 101, no. 23, 8 juin 2004, pages 8786-8790) décrit l'utilisation du rolipram pour les lésions associées à la moelle épinière.

Il existe un besoin de trouver des médicaments permettant de traiter les patients souffrant de traumatismes de la moelle épinière. Des études chez l'animal ont montré qu'une voie possible est l'administration de composés inhibant les phosphodiesterases 4 (PDE 4), tels que par exemple le rolipram. Toutefois, des études cliniques ont montré que ce composé, ainsi que d'autres inhibiteurs de PDE 4, induit des effets émétisants qui ne permettent pas son utilisation en thérapie.

On a trouvé maintenant que le 4-cyclopropylméthoxy-*N*-(3,5-dichloro-1-oxydo-pyridin-4-yl)-5-(méthoxy)pyridine-2-carboxamide peut être utilisé dans le traitement des traumatismes de la moelle épinière, tout en évitant les effets émétisants à des doses thérapeutiques acceptables.

Un premier objet de l'invention concerne donc l'utilisation du 4-cyclopropylméthoxy-*N*-(3,5-dichloro-1-oxydo-pyridin-4-yl)-5-(méthoxy)pyridine-2-carboxamide pour la préparation d'un médicament destiné au traitement des traumatismes de la moelle épinière.

Selon un mode d'exécution de l'invention, l'utilisation du 4-cyclopropylméthoxy-*N*-(3,5-dichloro-1-oxydo-pyridin-4-yl)-5-(méthoxy)pyridine-2-carboxamide peut se faire sous forme de base ou de sel d'addition à un acide.

Les sels utilisables dans le cadre de l'invention peuvent être préparés avec des acides pharmaceutiquement acceptables, mais les sels d'autres acides utiles, par exemple, pour la purification ou l'isolement du 4-cyclopropylméthoxy-*N*-(3,5-dichloro-1-oxydo-pyridin-4-yl)-5-(méthoxy)pyridine-2-carboxamide, font également partie de l'invention.

L'utilisation du 4-cyclopropylméthoxy-*N*-(3,5-dichloro-1-oxydo-pyridin-4-yl)-5-(méthoxy)pyridine-2-carboxamide selon l'invention peut se faire également sous forme d'hydrate ou de solvate. Par hydrate ou solvate, on entend l'association ou la combinaison de une ou plusieurs molécules de 4-cyclopropylméthoxy-*N*-(3,5-dichloro-1-oxydo-pyridin-4-yl)-5-(méthoxy)pyridine-2-carboxamide avec une ou plusieurs molécules d'eau ou de solvant.

Au sens de la présente invention, on entend par traumatisme de la moelle épinière les pathologies aiguës ou chroniques qui ont une origine externe et qui détruisent les tractus et/ou les neurones spinaux, et qui surviennent par exemple lors d'une chute, un choc, d'un écrasement ou d'un accident de la circulation.

Un second objet de l'invention concerne une composition pharmaceutique comprenant, à titre de principe actif, le 4-cyclopropylméthoxy-*N*-(3,5-dichloro-1-oxydo-pyridin-4-yl)-5-(méthoxy)pyridine-2-carboxamide, et un ou plusieurs excipients pharmaceutiquement acceptables.

La composition utilisée selon l'invention comprend une dose efficace du principe actif.

Par exemple, les doses journalières de principe actif utilisable selon l'invention sont de 0,001 à 10 mg/jour.

Selon la pratique habituelle, le dosage approprié à chaque patient est déterminé par le médecin selon le mode d'administration, l'âge, le poids et la réponse dudit patient.

Les doses dépendent de l'effet recherché, de la durée du traitement et de la voie d'administration utilisée.

Il peut y avoir des cas particuliers où des dosages plus élevés ou plus faibles sont appropriés. De tels dosages ne sortent pas du cadre de l'invention.

Les excipients sont choisis selon la forme pharmaceutique et le mode d'administration souhaité, parmi les excipients habituels qui sont connus de l'homme du métier.

La composition peut être administrée par voie orale, parentérale (y compris intrathécale), ou rectale.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intra musculaire, intraveineuse ou intrathécale, les formes d'administration rectale

2

et les implants. Pour l'application topique, on peut utiliser les principes actifs selon l'invention dans des crèmes, gels, pommades ou lotions.

Lorsqu'on prépare une composition sous forme de comprimé, on mélange le principe actif avec un ou plusieurs excipients pharmaceutiques, tels que la gélatine, l'amidon, le lactose, le stéarate de magnésium, le talc, la silice, la gomme arabique, le mannitol, la cellulose microcristalline, l'hypromellose ou analogues.

On peut enrober les comprimés de saccharose, d'un dérivé cellulosique ou d'autres matières adaptées à l'enrobage. Les comprimés peuvent être réalisés par différentes techniques, telles que la compression directe, la granulation sèche ou humide ou la fusion à chaud.

On peut également obtenir une composition pharmaceutique sous forme de gélule en mélangeant le principe actif avec un diluant et en versant le mélange obtenu dans des gélules molles ou dures.

Pour une administration parentérale, on utilise des suspensions aqueuses, des solutions salines isotoniques ou des solutions stériles et injectables qui contiennent des agents pharmacologiquement compatibles, par exemple le propylèneglycol ou le butylèneglycol.

A titre d'exemple, une forme unitaire d'administration de 4-cyclopropylméthoxy-*N*-(3,5-dichloro-1-oxydo-pyridin-4-yl)-5-(méthoxy)pyridine-2-carboxamide sous forme de comprimé comprend les ingrédients suivants :

| | |
|---|---|
| 4-cyclopropylméthoxy-*N*-(3,5-dichloro-1-oxydo-pyridin-4-yl)-5-(méthoxy)pyridine-2-carboxamide | 1 mg |
| Mannitol | 224 mg |
| Croscarmellose sodique | 5 mg |
| Amidon de maïs | 15 mg |
| Hydroxypropyl-méthylcellulose | 2 mg |
| Stéarate de magnésium | 3 mg |

Les effets du 4-cyclopropylméthoxy-*N*-(3,5-dichloro-1-oxydo-pyridin-4-yl)-5-(méthoxy)pyridine-2-carboxamide utilisé selon l'invention ont été évalués dans un modèle de traumatisme spinal chez la souris en utilisant le test de la barre (P. Barnéoud, NeuroReport 1997, 8, 2861-2865).

EXEMPLE 1 : Evaluation de l'efficacité du 4-cyclopropylméthoxy-*N*-(3,5-dichloro-1-oxydo-pyridin-4-yl)-5-(méthoxy)pyridine-2-carboxamide dans le traitement des traumatismes de la moelle épinière :

Le test de Beam Balance consiste à placer la souris à l'extrémité d'une barre horizontale de 30 cm de longueur et 1,5 cm de largeur, surélevée de 20 cm par rapport au sol. On mesure le temps nécessaire pour que la souris rejoigne l'extrémité opposée de la poutre. Le test est arrêté au bout de 12 secondes. Si l'animal chute ou n'accomplit pas l'épreuve, on note le temps maximum.

Des souris femelles OF1 (Iffa Credo Lyon, France) de 12 à 14 g et âgées de 34 semaines sont placées dans des cages d'expérimentation (32 X 21 X 14 cm) pourvues en nourriture et en eau à volonté, à la température contrôlée de $22\pm1°C$.

Une expérience mettant en oeuvre le test de Beam Balance est menée pour évaluer l'efficacité du 4-cyclopropylméthoxy-*N*-(3,5-dichloro-1-oxydo-pyridin-4-yl)-5-(méthoxy)pyridine-2-carboxamide, dans le traitement du traumatisme de la moelle épinière.

Les souris sont soumises à un préapprentissage qui leur permet de se familiariser avec le test d'évaluation et leur permet d'atteindre un niveau de performance optimale et identique.

Les animaux sont ensuite partagés en trois groupes, puis un traumatisme est induit de manière contrôlée chez deux des trois groupes de souris avant le début de la phase de test.

Ce traumatisme consiste en une lésion de la moelle épinière localisée au niveau de la vertèbre thoracique Th8. La lésion est générée par 3 cycles de congélation-décongélation successifs en appliquant de l'azote liquide.

Les conséquences fonctionnelles du traumatisme sont alors mesurées aux jours 2, 7, 14 et 21 ainsi qu'au jour 28.

Les groupes suivants ont été constitués :

- Le groupe 1 (sans traumatisme) est constitué d'animaux contrôles qui ne sont soumis à aucun traumatisme.
- Le groupe 2 (traumatisme seul) est constitué d'animaux traumatisés auxquels on administre une dose par jour de véhicule (méthylcellulose (MC) (0,6 %) + tween-80 (0,5%) dans l'eau).
- Le groupe 3 (traumatisme + p.a. 0,01 mg/kg à +4 heures) reçoit une solution contenant du 4-cyclopropylméthoxy-*N*-(3,5-dichloro-1-oxydo-pyridin-4-yl)-5-(méthoxy)pyridine-2-carboxamide (0,01 mg/kg) dans le véhicule (MC (0,6 %) + tween-80 (0,5 %) dans l'eau) par voie orale 4 heures après la lésion, puis journellement par voie orale pendant 4 semaines après le traumatisme.

Les résultats obtenus pour chaque groupe de souris sont rapportés dans le tableau 1 :

Tableau 1 : Résultats au test de la barre des groupes 1 à 3.

| Groupes | Résultats (secondes) | | | | | | |
|---|---|---|---|---|---|---|---|
| | J-4 | J=0 Traumatisme | J+2 | J+7 | J+14 | J+21 | J+28 |
| 1 (sans traumatisme) | 3,95 | Non | 3,52 | 3,77 | 3,25 | 2,99 | 2,92 |
| 2 (traumatisme seul) | 3,67 | Oui | 12,11 | 10,60 | 9,58 | 8,33 | 8,11 |
| 3 (traumatisme + p.a. à +4h). | 3,93 | Oui | 10,71 | 8,67 | 6,35 | 6,17 | 5,18 |

Le tableau 1 montre que les animaux traumatisés auxquels on a administré de façon curative le 4-cyclopropylméthoxy-*N*-(3,5-dichloro-1-oxydo-pyridin-4-yl)-5-(méthoxy)pyridine-2-carboxamide (groupe 3) rejoignent plus rapidement l'extrémité de la barre que les animaux traumatisés auxquels ce composé n'a pas été administré (groupe 2).

Dans l'ensemble, ces expériences montrent que les animaux traumatisés auxquels on a administré le 4-cyclopropylméthoxy-*N*-(3,5-dichloro-1-oxydo-pyridin-4-yl)-5-(méthoxy)pyridine-2-carboxamide présentent un meilleur rétablissement dans les fonctions motrices, que les animaux traumatisés non traités. Les résultats sont similaires lorsque l'invention est administrée en traitement préventif, c'est-à-dire préalablement au traumatisme.

EXEMPLE comparatif 2: Evaluation de l'efficacité du ((4R)-4-r3-(Cyclopentyloxy)-4-méthoxyphényl]pyrrolidine-2 -one) dans le traitement des traumatismes de la moelle épinière :

Une expérience similaire à celle de l'exemple 1 a été menée en administrant à des souris OF1 (Charles River, France) de 12 à 14 g et âgées de 34 semaines du ((4R)-4-[3-(Cyclopentyloxy)-4-méthoxyphényl]pyrrolidine-2-one), dans le traitement des traumatismes de la moëlle épinière.

Le ((4R)-4-[3-(Cyclopentyloxy)-4-méthoxyphényl]pyrrolidine-2-one), appelé également (*R*)-(-)-Rolipram, est notamment décrit dans le document US 4,193,926.

Les souris ont été testées dans le test de Beam Balance, selon des conditions opératoires identiques à celles décrites précédemment.

Les groupes suivants ont été constitués :

- Le groupe A (sans traumatisme) est constitué d'animaux contrôles qui ne sont soumis à aucun traumatisme.
- Le groupe B (traumatisme seul) est constitué d'animaux traumatisés auxquels on administre une dose par jour de véhicule (PEG200 2%).
- Le groupe C (traumatisme + p.a. 0,03 mg/kg à + 4 heures) reçoit une solution contenant du (*R*)-(-)-rolipram (0,03 mg/kg) dans le véhicule (PEG200 2%) par voie orale 4 heures et 6 heures après la lésion, puis journellement par voie orale pendant 4 semaines après le traumatisme.

Les résultats obtenus pour chaque groupe de souris sont exprimés en pourcentage (%) de déficit des fonctions motrices chez les souris traumatisées par rapport aux souris non traumatisées :

Pour ce faire, on mesure la différence de temps réalisé entre le groupe de souris testées auxquelles on a administré le (*R*)-(-)-Rolipram (groupes C) et le groupe de souris non traumatisées (groupe A), que l'on exprime en pourcentage par rapport à la différence de temps réalisé entre les souris traumatisées auxquelles on a administré une dose par jour de véhicule (groupe B) et le temps réalisé par les souris non traumatisées (groupe A). Ce rapport donne donc le pourcentage de déficit des fonctions motrices des souris traumatisées par rapport aux souris non traumatisées.

Ce calcul du pourcentage de déficit des souris testées est réalisé selon la formule suivante :

$$\text{\% de déficit du groupe (C) = [(moyenne du temps de parcours du groupe C)-(moyenne du temps de parcours du groupe A)] / [(moyenne du temps de parcours du groupe B)-(moyenne du temps de parcours du groupe A)]}$$

avec :

Groupe A : animaux non traumatisés
Groupe B : animaux traumatisés traités au véhicule
Groupe C : animaux traumatisés traités au (*R*)-(-)-Rolipram

Plus le pourcentage exprimé est élevé, plus le déficit des fonctions motrices constaté est important. Ainsi, un résultat de 100% (cent pourcent) correspond à un groupe de souris traumatisées sur lesquelles on ne constate aucun effet thérapeutique.
Une valeur supérieure à 100% indique que le groupe de souris évaluées a réalisé, en moyenne, le parcours dans un temps supérieur à celui réalisé, en moyenne, par le groupe de souris traumatisées traitées au véhicule.
Les résultats obtenus pour chaque groupe de souris sont rapportés dans le tableau 2 :

Tableau 2 : Résultats au test de la barre des groupes A à C :

| Groupes | Temps de parcours (% de déficit des fonctions motrices) | | | | | | |
|---|---|---|---|---|---|---|---|
| | J=0 Traumatisme | J+2 | J+7 | J+14 | J+21 | J+28 | Moyenne |
| A: (sans traumatisme) | Non | 0 | 0 | 0 | 0 | 0 | 0 |
| B: (traumatisme seul) | Oui | 100 | 100 | 100 | 100 | 100 | 100 |
| C: (traumatisme + (*R*)-(-)-rolipram (0,03 mg/kg po à +4h)) | Oui | 110 | 67 | 48 | 122 | 108 | 91 |

Ces expériences montrent que les animaux traumatisés traités au (*R*)-(-)-Rolipram, même à des doses administrées 3 fois plus importantes que les doses administrées de 4-cyclopropylméthoxy-*N*-(3,5-dichloro-1-oxydo-pyridin-4-yl)-5-(méthoxy)pyridine-2-carboxamide, présentent un déficit des fonctions motrices qui est supérieur à celui des animaux traités au 4-cyclopropylméthoxy-*N*-(3,5-dichloro-1-oxydo-pyridin-4-yl)-5-(méthoxy)pyridine-2-carboxamide.
A titre de comparaison, les valeurs obtenues pour les souris traumatisées auxquelles on a administré le 4-cyclopropylméthoxy-*N*-(3,5-dichloro-1-oxydo-pyridin-4-yl)-5-(méthoxy)pyridine-2-carboxamide (groupe 3 du tableau 1) sont rapportées dans le tableau 3 sous forme de pourcentage de déficit des fonctions motrices :

Tableau 3 : Comparaison des résultats obtenus pour les groupes de souris 3 et C.

| Groupes | Temps de parcours (% de déficit) | | | | | | |
|---|---|---|---|---|---|---|---|
| | J=0 Traumatisme | J+2 | J+7 | J+14 | J+21 | J+28 | Moyenne |
| C: (traumatisme + (R)-(-)-rolipram (0,03 mg/kg po à +4h)) | Oui | 110 | 67 | 48 | 122 | 108 | 91 |
| 3: (traumatisme + composé de l'invention (0,01 mglkg po à +4h)) | Oui | 84 | 72 | 49 | 60 | 44 | 62 |

Groupe C : animaux traumatisés traités au (*R*)-(-)-Rolipram.
Groupe 3: animaux traumatisés traités au 4-cyclopropylméthoxy-*N*-(3,5-dichloro-1-oxydo-pyridin-4-yl)-5-(méthoxy)pyridine-2-carboxamide.

Les animaux traités au 4-cyclopropylméthoxy-N-(3,5-dichloro-1-oxydo-pyridin-4-yl)-5-(méthoxy)pyridine-2-carboxamide présentent un taux de déficit des fonctions motrices qui est inférieur à celui des animaux traités au (R)-(-)-Rolipram.

EXEMPLE 3: Evaluation des effets émétisants du 4-cyclopropylméthoxy-N-(3,5-dichloro-1-oxydo-pyridin-4-yl)-5-(méthoxy)pyridine-2-carboxamide.

Le pouvoir émétique du 4-cyclopropylméthoxy-N-(3,5-dichloro-1-oxydo-pyridin-4-yl)-5-(méthoxy)pyridine-2-carboxamide a été évalué chez le furet. Deux groupes de furets ont été utilisés, le premier recevant le véhicule (PEG200) et le second le 4-cyclopropylméthoxy-N-(3,5-dichloro-1-oxydo-pyridin-4-yl)-5-(méthoxy)pyridine-2-carboxamide en solution dans le véhicule (PEG 200), par gavage oral, aux doses de 0,05 mg/kg et de 0,1 mg/kg. Les animaux ont été observés continuellement pendant les 2 heures suivant l'administration puis une fois par heure jusqu'à 6 heures après l'administration. Les signes cliniques (en particulier hauts-le-coeur et vomissements) ont été notés.

Administré à 0,1 mg/kg, le 4-cyclopropylméthoxy-N-(3,5-dichloro-1-oxydo-pyridin-4-yl)-5-(méthoxy)pyridine-2-carboxamide n'induit aucun haut-le-coeur ni vomissement chez les 5 furets traités.

Ces résultats montrent que l'administration d'une dose thérapeutique de 4-cyclopropylméthoxy-N-(3,5-dichloro-1-oxydo-pyridin-4-yl)-5-(méthoxy)pyiridine-2-carboxamide pour traiter des traumatismes de la moëlle épinière, n'entraîne pas d'effet émétique.

EXEMPLE comparatif 4: Evaluation des effets émétisants du (R)-(-)-rolipram ( ((4R)-4-[3-(Cyclopentyloxy)-4-methoxyphenyl]pyrrolidine-2-one))

Le pouvoir émétique du (R)-(-)-rolipram a été évalué chez le furet. Deux groupes de furets ont été utilisés, le premier recevant le véhicule (PEG200) et le second le (R)-(-)-rolipram en solution dans le véhicule (PEG 200), par gavage oral, aux doses de 0,05 mg/kg et de 0,1 mg/kg. Les animaux ont été observés continuellement pendant les 2 heures suivant l'administration puis une fois par heure jusqu'à 6 heures après l'administration. Les signes cliniques ont été notés.

Administré à 0,05 mg/kg et 0,1 mg/kg, le (R)-(-)-rolipram induit des vomissements chez les furets traités.

Les résultats des exemples 3 et 4 montrent que l'administration d'une dose thérapeutique de (R)-(-)-Rolipram entraîne des effets émétiques.

Ainsi, le 4-cyclopropylméthoxy-N-(3,5-dichloro-1-oxydo-pyridin-4-yl)-5-(méthoxy)pyridine-2-carboxamide est utile dans la préparation d'un médicament pour le traitement des traumatismes du système nerveux, notamment les traumatismes de la moelle, comme par exemple les traumatismes survenant lors d'une chute, un choc ou un accident automobile ou les traumatismes cérébraux, tout en évitant d'éventuels effets émétisants.

## Revendications

1. Utilisation du 4-cyclopropylméthoxy-N-(3,5-dichloro-1-oxydo-pyridin-4-yl)-5-(méthoxy)pyridine-2-carboxamide sous forme d'hydrate, de solvate, de base ou de sel d'addition à un acide, pour la préparation d'un médicament destiné au traitement des traumatismes de la moelle épinière.

2. Utilisation selon la revendication 1, **caractérisée en ce que** le 4-cyclopropylméthoxy-N-(3,5-dichloro-1-oxydo-pyridin-4-yl)-5-(méthoxy)pyridine-2-carboxamide est sous forme de base.

## Claims

1. Use of 4-cyclopropylmethoxy-N-(3,5-dichloro-1-oxidopyridin-4-yl)-5-(methoxy)pyridine-2-carboxamide in the form of a hydrate, of a solvate, of a base or of an addition salt with an acid, for the preparation of a medicament for use in the treatment of spinal cord traumas.

2. Use according to Claim 1, **characterized in that** the 4-cyclopropylmethoxy-N-(3,5-dichloro-1-oxido-pyridin-4-yl)-5-(methoxy)pyridine-2-carboxamide is in the form of a base.

**Patentansprüche**

1. Verwendung von 4-Cyclopropylmethoxy-*N*-(3,5-dichlor-1-oxidopyridin-4-yl)-5-(methoxy)pyridin-2-carboxamid in Hydrat-, Solvat-, Basen- oder Säureadditionssalzform zur Herstellung eines Arzneimittels zur Behandlung von Rükkenmarkstraumen.

2. Verwendung nach Anspruch 1, **dadurch gekennzeichnet, daß** das 4-Cyclopropylmethoxy-*N*-(3,5-dichlor-1-oxidopyridin-4-yl)-5-(methoxy)pyridin-2-carboxamid in Basenform vorliegt.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 9504045 A
- GB 2406856 A
- US 2007021451 A
- US 4193926 A

**Littérature non-brevet citée dans la description**

- **Nikulina Elena et al.** The phosphodiesterase inhibitor rolipram delivered after a spinal cord lesion promotes axonal regeneration and functional recovery. *PROCEEDINGS OF THE NATIONAL ACADEMY OF SCIENCES OF THE UNITED STATES OF AMERICA 08 JUN 2004 UNITED STATES,* 08 Juin 2004, vol. 101 (23), 8786-8790
- **P. Barnéoud.** *NeuroReport,* 1997, vol. 8, 2861-2865